# DEMANDE DE BREVET EUROPEEN

(11) **EP 3 076 156 A1**
(43) Date de publication de la demande: **05.10.2016**
(21) Numéro de dépôt: 16162804.5
(22) Date de dépôt: 30.03.2016
(51) Int. Cl.: G01N 15/00, G01N 33/49, G01N 15/14

(54) **PROCÉDÉ DE DÉTERMINATION DU NIVEAU D AGGLUTINATION DE PARTICULES DANS UN ÉCHANTILLON**

(30) Priorité: 31.03.2015 FR 1552715
(71) Demandeur: Commissariat à l'Énergie Atomique et aux Énergies Alternatives, 75015 Paris (FR)
(72) Inventeur: Huet, Maxime, 38000 GRENOBLE (FR); Coutard, Jean-Guillaume, 38660 SAINT-PANCRASSE (FR)
(74) Mandataire: GIE Innovation Competence Group

(57) **Abrégé**

L'invention concerne un procédé pour quantifier le niveau d'agglutination de particules dans un échantillon, en particulier un échantillon biologique, et notamment du sang. L'échantillon biologique est disposé entre une source de lumière et un photodétecteur matriciel. L'image acquise par le photodétecteur est représentative du niveau d'agglutination des particules dans l'échantillon. La source de lumière émet une onde lumineuse dont la bande spectrale s'étend entre 400 et 600 nm, ce qui constitue un optimum entre une absorption trop faible et une absorption trop élevée, compte tenu de l'épaisseur de l'échantillon.

## Description

### DOMAINE TECHNIQUE

L'invention concerne un procédé pour déterminer un niveau d'agglutination de particules dans un échantillon, et en particulier une agglutination de cellules, notamment des cellules sanguines, à l'aide d'un dispositif optique. Le procédé peut être mis en oeuvre à des fins de diagnostic médical.

### ART ANTERIEUR

L'utilisation de méthodes optiques pour observer l'agglutination de cellules dans un liquide, et pour en tirer des informations quantitatives, par exemple à des fins de diagnostic, est récente.

Le brevet européen EP2669678 décrit un procédé permettant la détermination d'un état d'agglutination de particules, et notamment des globules rouges, en disposant un échantillon, comportant les particules, entre une source de lumière et un photodétecteur matriciel. L'échantillon comporte un réactif apte à favoriser l'agglutination des globules rouges. Les globules rouges sont éclairés par la source de lumière, et génèrent, au niveau du détecteur, une image représentative de leur état d'agglutination. Plus précisément, au fur et à mesure que les globules rouges s'agglutinent, la morphologie de l'image détectée évolue. Ainsi, par une analyse d'image, il est possible de suivre l'évolution de l'agglutination des globules rouges dans l'échantillon. Il est également possible d'établir des indicateurs quantitatifs caractérisant l'état d'agglutination, c'est-à-dire la quantité de globules rouges agglutinés dans l'échantillon.

Ce procédé s'avère particulièrement efficace pour réaliser des analyses fiables à l'aide d'un moyen simple et peu onéreux, pouvant par exemple être intégré dans un dispositif portable, désigné par le terme anglais « Point of Care », signifiant « au chevet du patient ». Il permet par exemple d'effectuer un groupage sanguin, ou un dosage d'analyte présent dans l'échantillon, notamment en introduisant un réactif bispécifique dans ce dernier, le réactif bispécifique étant apte à se lier conjointement à l'analyte et à un globule rouge. Ainsi, lorsque la quantité de réactif bispécifique introduite dans l'échantillon est maîtrisée, la formation d'agglutinats de globules rouges dépend de la concentration dudit analyte dans l'échantillon. La détermination d'un indicateur quantifiant l'agglutination permet un dosage de la concentration de l'analyte.

La demande de brevet européen EP0637744 décrit un procédé de détermination d'un niveau d'agglutination de globules rouges dans un échantillon. L'échantillon est éclairé par une source de lumière, un filtre optique passe bande étant disposé entre l'échantillon et un photodétecteur matriciel. L'échantillon est disposé dans une enceinte, comportant un étage filtrant composé de billes de verre. L'échantillon subit une centrifugation, permettant une séparation entre des agglutinats de globules rouges et des globules rouges non agglutinées. Au cours de cette centrifugation, les agglutinats sont bloqués par l'étage filtrant, tandis que les globules non agglutinés passent à travers ce dernier. Le procédé requiert une centrifugation ainsi qu'une chambre fluidique complexe, comportant un étage filtrant apte à retenir les agglutinats de globules rouges tout en laissant passer les globules rouges non agglutinés. On ne peut alors pas parler d'un procédé simple à mettre en oeuvre.

Les inventeurs ont souhaité apporter un perfectionnement au procédé décrit dans EP2669678, en améliorant notamment sa sensibilité tout en conservant une simplicité de mise en oeuvre.

### EXPOSE DE L'INVENTION

Un objet de l'invention est un procédé de détermination d'un niveau d'agglutination dans un échantillon, l'échantillon comportant des particules, lesdites particules étant aptes à s'agglutiner de manière à former un ou une pluralité d'agglutinats dans ledit échantillon, le procédé comportant les étapes suivantes :
- illumination dudit échantillon à l'aide d'une source de lumière, la source de lumière produisant une onde lumineuse incidente, se propageant vers l'échantillon selon un axe de propagation,
- acquisition, à l'aide d'un photodétecteur matriciel d'une image de l'échantillon, l'échantillon étant disposé entre ladite source de lumière et ledit photodétecteur matriciel,
- détermination d'un niveau d'agglutination de particules dans l'échantillon, à l'aide de
   ladite image,
caractérisé en ce que :
- ladite onde lumineuse incidente présente une bande spectrale centrée sur une longueur d'onde, dite longueur d'onde centrale, inférieure à 600 nm.

Par niveau d'agglutination, on entend une valeur représentative de la quantité de particules agglutinées dans l'échantillon.

Les particules peuvent notamment être des globules rouges.

L'échantillon peut comporter un liquide corporel, ce dernier étant notamment du sang. L'échantillon peut être du sang total.

L'épaisseur de l'échantillon, selon l'axe de propagation, peut notamment être inférieure à 1 cm, voire inférieure à 5 mm, voire, et en particulier lorsque l'échantillon est du sang total, inférieure à 1 mm voire à 500 µm.

La bande spectrale est de préférence centrée sur une longueur d'onde, dite longueur d'onde centrale, comprise entre 400 nm et 600 nm. De préférence, ladite bande spectrale ne s'étend ni en dessus de 600 nm, ni en dessous de 400 nm.

De préférence, la largeur de ladite bande spectrale est inférieure à 100nm, et de préférence à 50 nm, voire à 25 nm.

L'estimation du niveau d'agglutination peut comporter les étapes suivantes :
i) sélection, dans ladite image, de zones sombres, dont l'intensité est inférieure à un premier seuil et/ou de zones claires, dont l'intensité est supérieure à un deuxième seuil,
ii) détermination d'un indicateur statistique représentatif desdites zones sélectionnées
iii) estimation d'un niveau d'agglutination en fonction de la valeur dudit indicateur. Selon un mode de réalisation, le procédé comporte une étape d'ajout d'un réactif dans l'échantillon, apte à engendrer l'agglutination desdites particules.

Selon un mode de réalisation, le procédé comporte une étape de dosage d'un analyte présent dans l'échantillon en fonction du niveau d'agglutination estimé.

Selon un mode de réalisation, l'échantillon comporte du sang et le procédé comporte une étape de détermination d'un groupe sanguin en fonction du niveau d'agglutination estimé.

Un autre objet de l'invention est un dispositif pour la détermination d'un niveau d'agglutination dans un échantillon, l'échantillon comportant des particules, lesdites particules étant aptes à s'agglutiner de manière à former un ou une pluralité d'agglutinats dans ledit échantillon, le dispositif comprenant :
- une source de lumière agencée pour produire une onde lumineuse incidente, selon un axe de propagation, en direction dudit échantillon,
- un support, pour maintenir l'échantillon entre ladite source de lumière et un photodétecteur matriciel,
- le photodétecteur matriciel, étant agencé pour acquérir une image de l'onde lumineuse transmise par l'échantillon, lorsque ce dernier est exposé à ladite onde lumineuse incidente,
- un calculateur, apte à déterminer un niveau d'agglutination des particules dans l'échantillon en fonction de ladite image,
le dispositif étant caractérisé en ce que :
- la source de lumière est configurée de telle sorte que ladite onde lumineuse incidente présente une bande spectrale centrée sur une longueur d'onde, dite longueur d'onde centrale, inférieure à 600 nm.

La bande spectrale est en particulier centrée sur une longueur d'onde comprise entre 440 nm et 600 nm. Sa largeur est de préférence inférieure à 100nm, et encore de préférence inférieure à 50 nm, voire 25 nm.

### FIGURES

La figure 1 représente un exemple de dispositif selon l'invention.
La figure 2 représente une courbe montrant l'absorption d'un échantillon de sang en fonction de la longueur d'onde.
La figure 3 représente le spectre de transmission d'un filtre mis en oeuvre dans un exemple de réalisation.
Les figures 4A, 4B et 4C représentent des images obtenues respectivement dans trois configurations d'essais différentes.
La figure 5 représente des profils d'intensité réalisés sur les images 4A, 4B et 4C.
La figure 6 représente les histogrammes de l'intensité des pixels des images représentées sur les figures 4A, 4B et 4C.

### EXPOSE DE MODES DE REALISATION PARTICULIERS

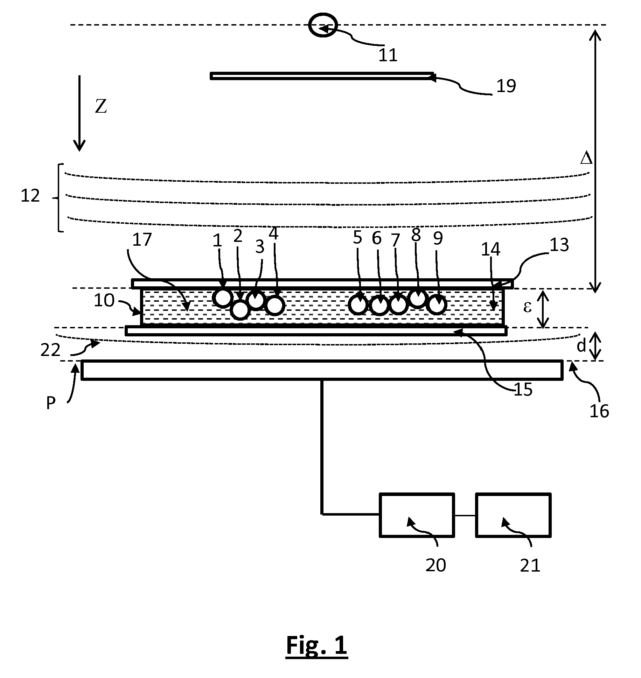

La figure 1 représente un exemple de dispositif objet de l'invention. Une source de lumière 11 est apte à produire une onde lumineuse 12, dite onde lumineuse incidente, en direction d'un échantillon 10, selon un axe de propagation Z.

L'échantillon 10 comporte un milieu 14 ainsi que des particules 1, 2, ...9, baignant dans ce milieu.

Le milieu 14 peut notamment comporter un liquide, en particulier un liquide corporel, par exemple du sang. Il peut notamment s'agir de sang total. Les particules 1,2..9 peuvent être des particules sanguines, et plus particulièrement des globules rouges.

La distance Δ entre la source de lumière et l'échantillon est de préférence supérieure à 1 cm. Elle est de préférence comprise entre 1 et 30 cm, typiquement 5 cm.

De préférence, la source de lumière, vue par l'échantillon, est considérée comme ponctuelle, mais cela n'est pas nécessaire. Le terme ponctuel désigne le fait que son diamètre (ou sa diagonale) doit être inférieure au cinquième, mieux au dixième de la distance entre l'échantillon et la source de lumière. Ainsi, la lumière parvient à l'échantillon sous la forme d'ondes planes, ou pouvant être considérées comme telles.

La source de lumière 11 peut être associée à un diaphragme 18 de façon à apparaître ponctuelle. L'ouverture du diaphragme est typiquement comprise entre 50 µm et 1mm, de préférence entre 50 µm et 500 µm.

La source de lumière 11 peut également être fibrée. Dans ce cas, une fibre optique s'étend entre une première extrémité, disposée face à une source de lumière, et collectant la lumière de cette dernière, et une deuxième extrémité, émettant la lumière vers l'échantillon. Dans ce cas, cette deuxième extrémité est considérée comme étant la source de lumière 11.

L'échantillon 10 est supporté par une enceinte, comportant un fond 15 et un couvercle 13. Les parois latérales de l'enceinte ne sont pas représentées. Cette enceinte forme un support de l'échantillon. Typiquement une enceinte est une chambre fluidique, dans laquelle l'échantillon est introduit, par exemple par capillarité. Dans l'exemple considéré, le fond 15 et le couvercle 13 sont constitués de 2 lames transparentes distantes de 100 µm. La distance entre le fond 15 et le couvercle 13, selon l'axe de propagation Z, correspond à l'épaisseur ε de l'échantillon. Cette dernière varie typiquement entre 20 µm et 1 cm, et est de préférence comprise entre 50 µm et 500 µm, par exemple 150 µm.

L'échantillon 10 est disposé entre la source de lumière 11 et un photodétecteur matriciel 16, ou capteur d'image, apte à établir une image I. Le photodétecteur matriciel s'étend selon un plan de détection P, de préférence parallèlement, ou sensiblement parallèlement au fond 15 de l'enceinte délimitant l'échantillon. Le terme sensiblement parallèlement signifie que les deux éléments peuvent ne pas être rigoureusement parallèles, une tolérance angulaire de quelques degrés, inférieure à 20° ou inférieure à 10° étant admise.

La source de lumière 11 peut être temporellement cohérente, mais cela n'est pas nécessaire. En effet, une source de lumière cohérente comme une diode laser peut induire un phénomène de diffraction sur des poussières ou rayures, situées sur le trajet de la lumière, entre la source 11 et le photodétecteur matriciel 16. Ces effets de diffraction, générés par des éléments diffractants exogènes, ne faisant pas partie de l'échantillon, peuvent induire un bruit au niveau de l'image I formée par le photodétecteur 16. Aussi, les inventeurs considèrent qu'une source non temporellement cohérente, telle une diode électroluminescente, ou LED (de l'anglais Light Emitting Diode) est préférable.

Un filtre 19 peut être disposé entre la source de lumière et l'échantillon, de manière à bloquer les longueurs d'onde en dehors d'une bande passante prédéterminée. L'utilisation d'un tel filtre est détaillée ci-après. Ainsi, l'onde lumineuse 12 incidente à l'échantillon 10 s'étend selon une bande spectrale réduite par rapport à la bande spectrale émise par la source de lumière 11.

Le photodétecteur matriciel 16 comporte une matrice de pixels, de type CCD (de l'anglais Charge Coupled Device) ou un CMOS (de l'anglais Complementary Metal-Oxyde Semiconductor). Les CMOS sont les photodétecteurs préférés, car la taille des pixels est plus faible, ce qui permet d'acquérir des images dont la résolution spatiale est plus favorable.

De préférence, le photodétecteur matriciel 16 comprend une matrice de pixels, au-dessus de laquelle est disposée une fenêtre de protection transparente. La distance entre la matrice de pixels et la fenêtre de protection est généralement comprise entre quelques dizaines de µm à 150 à 200 µm. De préférence, le plan de détection P selon lequel s'étend le photodétecteur matriciel est perpendiculaire à l'axe de propagation Z de l'onde lumineuse incidente (12).

D'une manière générale, et cela quel que soit le mode de réalisation, la distance d entre l'échantillon et les pixels du photodétecteur est inférieure à 2 cm, voire à 1 cm, et préférentiellement comprise entre 50 µm et 2 cm, de préférence comprise entre 100 µm et 2 mm.

Les photodétecteurs matriciels dont le pas inter pixel est inférieur à 3 µm sont préférés, car ils permettent d'obtenir des images avec une résolution spatiale satisfaisante.

On remarque l'absence d'optique de grossissement entre le photodétecteur matriciel 16 et l'échantillon 10. Cela n'empêche pas la présence éventuelle de microlentilles de focalisation au niveau de chaque pixel du photodétecteur 16.

Le photodétecteur matriciel est apte à réaliser une image I d'une onde lumineuse 22 transmise par l'échantillon 10 lorsque ce dernier est éclairé par la source de lumière 11. L'image I est acquise à tout ou partie des longueurs d'onde produites par la source de lumière. Cette onde lumineuse 22 résulte de l'interaction des particules présentes dans l'échantillon avec l'onde lumineuse incidente 12, produite par la source de lumière 11. En effet, sous l'effet de l'onde lumineuse incidente, une particule de l'échantillon peut engendrer une onde diffractée, susceptible de produire une interférence avec l'onde incidente 12 traversant l'échantillon. L'interférence entre l'onde diffractée par la particule et l'onde lumineuse incidente 12 donne lieu, sur l'image acquise par le photodétecteur, à une figure de diffraction élémentaire, comportant une zone centrale et plusieurs anneaux de diffraction concentriques.

Par ailleurs, les particules présentes de l'échantillon peuvent s'agglutiner entre elles, de façon spontanée ou sous l'effet d'un réactif 17 ajouté à l'échantillon. Dans un tel cas, les agglutinats formés absorbent une partie de l'onde lumineuse incidente 12.

Ainsi, l'onde lumineuse 22 à laquelle est exposé le photodétecteur matriciel, peut comprendre :
- une composante résultant de la diffraction de particules présentes dans l'échantillon, sous l'effet de l'onde lumineuse incidente 12, cette composante de diffraction se traduisant par la présence de figures de diffraction élémentaires sur le photodétecteur, chaque figure de diffraction élémentaire étant associée à une particule diffractante ;
- une composante résultant de l'absorption de l'onde lumineuse incidente dans l'échantillon, cette composante d'absorption se traduisant par une diminution de l'intensité lumineuse au niveau du détecteur.

Un processeur 20, par exemple un microprocesseur, est apte à traiter les images I générées par le photodétecteur matriciel 16. En particulier, le processeur est un microprocesseur relié à une mémoire programmable 21 dans laquelle est stockée une séquence d'instructions pour effectuer les opérations de traitement d'image et de calcul décrites dans cette description.

Ce dispositif est similaire à celui décrit dans le brevet européen EP2669678, précédemment commenté. Cependant, les exemples décrits dans ce dernier mentionnent l'utilisation d'une source de lumière dont la bande spectrale d'émission est centrée sur la longueur d'onde λ = 670 nm. En effet, il est connu que le spectre d'absorption du sang, et en particulier de l'oxyhémoglobine, présente un plateau au-delà de 600 nm, l'absorption étant particulièrement faible dans une bande spectrale comprise entre 600nm et 900 nm, soit dans le domaine du rouge ou du proche infrarouge.

De ce fait, dans les exemples décrits dans le brevet européen précité, la composante issue de la diffraction des particules est probablement prédominante, du fait de l'utilisation d'une source de lumière de longueur d'onde d'émission à l'égard de laquelle l'absorption du sang est faible.

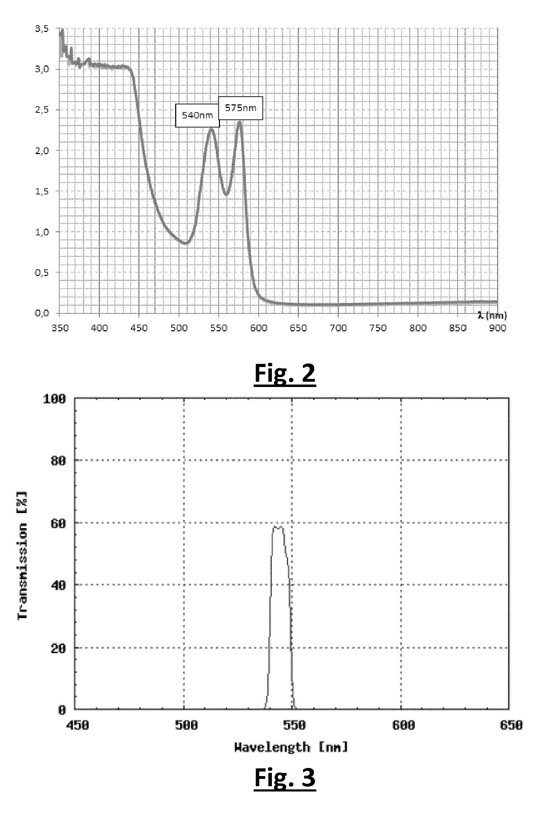

La figure 2 représente le spectre d'absorption d'un échantillon composé de sang total auquel une concentration de Saponine de 25mg/ml a été ajoutée, l'échantillon étant disposé dans une chambre fluidique transparente d'épaisseur ε égale à 150 µm. L'axe des abscisses représente la longueur d'onde, tandis que l'axe des ordonnées représente l'absorption mesurée.

De façon connue, l'ajout de Saponine entraîne la lyse des globules rouges dans l'échantillon, désignée par le terme hémolyse. Cela permet de limiter l'effet de la diffusion de la lumière par les globules rouges sur la mesure. Ces mesures ont été réalisées selon une configuration similaire à celle représentée dans la figure 1, en éclairant l'échantillon à l'aide d'une source de lumière blanche disposée en amont d'un monochromateur, le photodétecteur étant un spectrophotomètre. Ce spectre correspond sensiblement au spectre d'absorption de l'oxyhémoglobine, tel que présenté dans la littérature, avec la présence de deux pics d'absorption caractéristiques, respectivement centrés sur les longueurs d'onde 540 nm et 575 nm.

De ce fait, lorsqu'on souhaite réaliser des mesures optiques sur du sang, selon une configuration en transmission, il semble logique de privilégier une longueur d'onde apte à être aisément transmise par l'échantillon, c'est-à-dire supérieure à 600 nm, et cela tout particulièrement lorsque l'épaisseur de l'échantillon est suffisamment faible.

Le terme configuration en transmission désigne le fait que l'échantillon analysé est disposé entre la source de lumière et le photodétecteur matriciel, ce dernier collectant le signal lumineux transmis par l'échantillon.

Cependant, les inventeurs, cherchant à perfectionner le procédé objet du brevet précité, ont estimé que pour réaliser des mesures représentatives du niveau d'agglutination de particules telles des globules rouges, il était envisageable, et même préférable, d'utiliser une source de lumière configurée de telle sorte que l'onde lumineuse 12 incidente à l'échantillon ait une longueur d'onde λ inférieure à 600 nm.

En particulier, la bande spectrale de l'onde lumineuse incidente 12 est de préférence comprise entre 400 nm et 600 nm, ou encore de préférence entre 450 nm et 600 nm, dans la mesure où l'épaisseur ε de l'échantillon permet une transmission suffisante du signal.

Ainsi, d'une façon générale, lorsqu'il s'agit de sang total, l'épaisseur ε de l'échantillon est de préférence inférieure à 1 mm, voire à 500 µm. La contrainte sur l'épaisseur ε est moindre lorsqu'il s'agit de sang dilué, ou lorsque la concentration en globules rouges est faible. Dans de tels cas, néanmoins, l'épaisseur ε de l'échantillon est de préférence inférieure à 1 cm.

Entre 400 nm et 600 nm, l'absorption du faisceau lumineux incident, émis par la source de lumière 11, est significative, sans pour autant être excessive.

Sont particulièrement préférées les bandes spectrales suivantes : 450 nm -480 nm ; 530 nm - 580 nm. En effet, comme on peut l'observer sur la figure 2, dans ces plages, la valeur de l'absorption du sang correspond à un compromis entre une absorption trop importante (par exemple en deçà de 440 nm) et une absorption trop faible (au-delà de 600 nm).

Aussi, d'une façon générale, la source de lumière 11 est configurée pour que la bande spectrale de l'onde lumineuse incidente 12 soit centrée sur une longueur d'onde λₑₘ, dite longueur d'onde centrale, inférieure à 600 nm, et de préférence comprise entre 400 et 600 nm, et encore de préférence comprise entre 440 et 600 nm, les plages spectrales [450 nm - 480 nm] et [530 nm -580 nm] étant considérées comme optimales.

De préférence, la bande spectrale de l'onde lumineuse incidente 12 a une largeur inférieure à 100nm, et encore de préférence inférieure à 50 nm ou, mieux, 25 nm. Par largeur de la bande spectrale, on entend la largeur à mi-hauteur du pic d'émission.

De préférence, la bande spectrale de l'onde incidente 12 ne s'étend ni en dessus de 600 nm, ni en dessous de 400 nm, ou éventuellement de façon marginale. Ainsi, au moins 80%, voire plus de 90% de l'intensité émise est située entre 400nm et 600 nm.

Cette bande spectrale de l'onde lumineuse incidente 12 est produite soit directement par la source de lumière 11, soit par un filtre 19 interposé entre la source de lumière et l'échantillon 10.

En l'absence d'agglutination de globules rouges, ou lorsque cette agglutination est négligeable, l'onde lumineuse incidente 12 est essentiellement absorbée et le signal optique recueilli au niveau du détecteur est faible. L'image I formée par ce dernier est une image sombre, homogène, comportant des pixels dont le niveau de gris est peu dispersé.

Lorsque l'agglutination devient significative, c'est-à-dire lorsqu'une quantité suffisante de globules rouges sont agglutinés, la concentration en globules rouges n'est plus homogène dans l'échantillon. Ce dernier se divise entre une partie appauvrie et une partie enrichie. La partie enrichie correspond à des zones, dites zones enrichies, de l'échantillon dans lesquelles la concentration en globules rouges augmente; il s'agit des agglutinats. La partie appauvrie correspond à des zones, dites zones appauvries de l'échantillon, dans lesquelles la concentration en globules rouges diminue, du fait de la formation des agglutinats.

On comprend alors que l'illumination de l'échantillon à l'aide d'un faisceau dont la longueur d'onde est significativement absorbée par les globules rouges se traduit, au niveau de l'image I formée sur le détecteur, par :
- des zones sombres A, dont le niveau de gris est faible, chaque zone sombre correspondant à la projection, sur le détecteur, d'une zone enrichie de l'échantillon, du fait de l'absorption du faisceau lumineux incident 12 par les agglutinats.
- des zones claires B, dont le niveau de gris est élevé, chaque zone claire correspondant à la projection, sur le détecteur, d'une zone appauvrie de l'échantillon, du fait de la transmission accrue du faisceau lumineux incident 12 entre les agglutinats.

Ainsi, plus la quantité de globules rouges agglutinés augmente, plus la segmentation de l'échantillon, entre zones appauvries et zones enrichies, s'accroît. L'image formée par le détecteur est représentative de cette segmentation, et apparaît de plus en plus contrastée. Le nombre de pixels inférieurs à un premier seuil, dit seuil bas, augmente, du fait de l'absorption du faisceau incident par les agglutinats de globules rouges, tandis que le nombre de pixels supérieurs à un deuxième seuil, dit seuil haut, supérieur au premier seuil, augmente également.

Des essais ont été réalisés, en disposant du sang total dans une chambre fluidique d'épaisseur ε égale à 150 µm. On a préalablement ajouté un réactif apte à favoriser l'agglutination de globules rouges. Il s'agit d'un réactif Anti-A issu d'un kit de typage sanguin, commercialisé par la société Diagast sous la référence Groupakit-70888, le dosage étant d'un volume de sang pour un volume de réactif.

La source de lumière a été disposée à une distance Δ = 5 cm de l'échantillon, ce dernier étant disposé à une distance d = 1mm du photodétecteur.

Le détecteur mis en oeuvre est un capteur CMOS monochrome 8 bits comportant 2592 x 1944 pixels, référence Mightex BTN-B050-U.

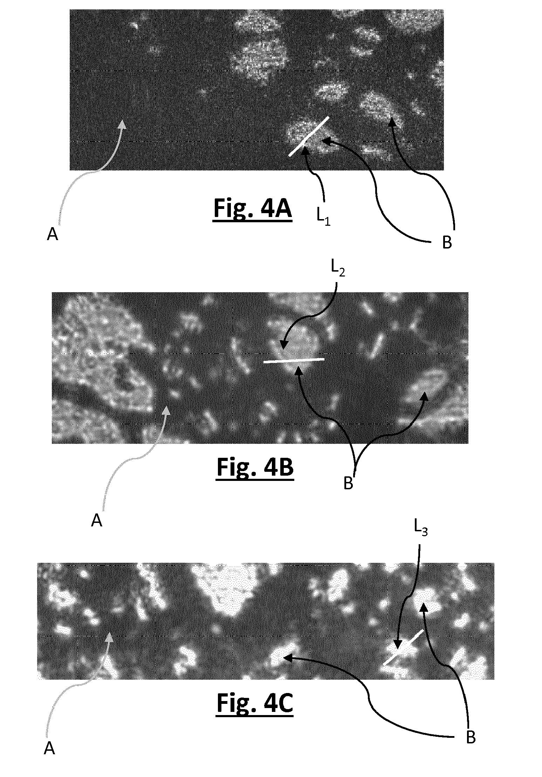

Lors du premier essai, la source de lumière 11 est une diode Laser émettant dans une bande spectrale centrée sur la longueur d'onde de 850 nm. L'image obtenue, 30 secondes après l'ajout du réactif, est représentée sur la figure 4A.

Lors du deuxième essai, la source de lumière 11 est une source de lumière blanche (Ocean optics halogen light source HL-2000-FHSA). L'image obtenue, 30 secondes après l'ajout du réactif, est représentée sur la figure 4B.

Lors du troisième essai, la source de lumière 11 est la source de lumière blanche utilisée dans l'essai précédent, un filtre 19 étant interposé entre la source de lumière et l'échantillon. Le filtre a pour objectif de réduire la bande d'émission de la source de lumière, en bloquant les longueurs d'onde en dehors d'une bande passante prédéterminée. La figure 3 représente la fonction de transmission de ce filtre, dont la référence est Omega optical filter XF1020 546DF10. La bande passante de ce filtre est centrée sur la longueur d'onde 546 nm, et s'étend sensiblement entre 538 nm et 552 nm. L'image obtenue, 30 secondes après l'ajout du réactif, est représentée sur la figure 4C.

On notera que les trois images respectivement représentées sur les figures 4A, 4B et 4C correspondent à des échantillons pouvant être considérés comme identiques : même concentration de réactif ajouté, même durée depuis l'ajout de ce dernier. Pourtant, l'image 4C apparaît nettement plus contrastée que l'image 4B, cette dernière apparaissant plus contrastée que l'image 4A.

Une image plus contrastée permet une meilleure distinction entre les zones claires et les zones sombres. Cela permet de mieux délimiter ces zones, notamment par seuillage d'intensité, en considérant les seuils bas et seuils haut précédemment cités, ou par des algorithmes de segmentation d'image. On obtient ainsi une grandeur statistique plus précise, permettant de quantifier le niveau d'agglutination de l'échantillon. La grandeur statistique peut être :
- une mesure de l'étendue spatiale des zones claires, zones regroupant les pixels dont l'intensité est supérieure audit seuil haut, l'étendue spatiale correspondant au nombre de pixels contenus dans cette zone ;
- une mesure de l'étendue spatiale des zones sombres, zones regroupant les pixels dont l'intensité est inférieure audit seuil bas ;
- des indicateurs statistiques relatifs à la distribution de l'intensité dans les zones claires et/ou dans les zones sombres : intensité moyenne, intensité médiane, intégrale de l'intensité, variance...

On comprend que plus l'image est contrastée, plus le niveau d'agglutination de l'échantillon est précisément quantifié.

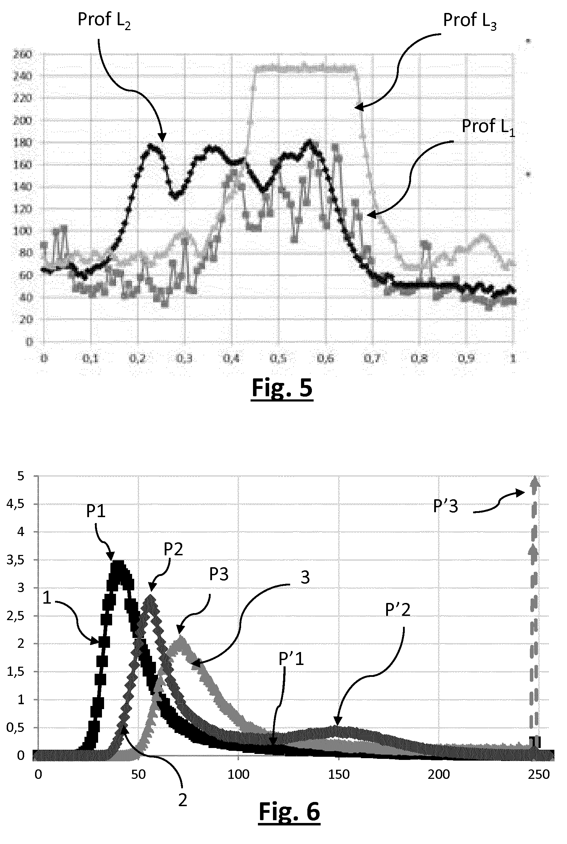

La figure 5 représente des profils d'intensité Prof L₁, Prof L₂ et Prof L₃ respectivement réalisés sur les lignes L₁, L₂ et L₃ respectivement représentées sur les figures 4A, 4B et 4C. Chaque profil a été réalisé de part et d'autre d'une zone claire B, ainsi que dans ladite zone claire, afin de pouvoir estimer un rapport signal sur bruit. On remarque que le rapport contraste sur bruit est nettement plus favorable sur le profil Prof L₃, obtenu sur l'image 4C réalisée avec une source de lumière blanche filtrée. Le rapport contraste sur bruit correspondant au profil Prof L₁, obtenu sur l'image 4A réalisée avec la source laser centrée sur λ=850 nm, est le plus faible.

Le terme rapport contraste à bruit s'entend comme la différence entre les intensités moyennes d'une zone claire et d'une zone sombre, divisée par l'écart type du bruit.

On comprend que l'invention représente un perfectionnement notable de configurations dans lesquelles la longueur d'onde de la source de lumière est supérieure à 600 nm, et cela au niveau du rapport signal sur bruit. La sensibilité est donc améliorée.

La figure 6 met en évidence l'effet de la source de lumière sur la distribution en intensité des pixels de l'image formée par le photodétecteur. Cette figure représente, pour chaque essai, l'histogramme d'intensité des pixels, le niveau d'intensité étant compris entre 0 (niveau de gris minimum - pixel noir) et 255 (niveau de gris maximum - pixel blanc), la dynamique de l'image I étant de 8 bits.

L'histogramme correspondant au premier essai (source laser, λ = 850 nm), portant la référence 1 sur la figure 6, comporte un pic principal P1 dont la valeur maximale correspond à un niveau d'intensité (ou de niveau de gris) de 40, et s'étendant entre les niveaux d'intensité 25 et 70. Ce pic correspond aux agglutinats, c'est-à-dire aux zones sombres A de la figure 4A. On discerne également une bosse P'1, de faible amplitude, s'étendant entre des valeurs d'intensité comprises entre environ 70 à environ 150, correspondant aux zones claires. Notons que les bornes de cette bosse ne sont pas nettes et sont difficiles à déterminer avec précision.

L'histogramme correspondant au deuxième essai (source de lumière blanche), portant la référence 2 sur la figure 6, comporte un pic principal P2 centré sur une valeur d'intensité de 70, et s'étendant entre les valeurs d'intensité 40 et d'environ 75 ainsi qu'un pic secondaire P'2, centré sur une valeur d'intensité proche de 150, et s'étendant entre les valeurs d'intensité de 120 à 180, ces valeurs étant indicatives, du fait de l'étalement de ce pic. P2 et P'2 correspondent respectivement aux agglutinats et aux zones claires. Précisions que des algorithmes de séparation de pics peuvent être appliqués, aboutissant à une délimitation plus précise de chaque pic.

Sur la base de cet histogramme, les zones sombres et les zones claires de l'image 4B peuvent être délimitées en considérant respectivement les pixels dont l'intensité est inférieure à 75 (seuil bas) et les pixels sont l'intensité est supérieure à 120 (seuil haut). L'écart entre le seuil bas et le seuil haut, exprimé en valeur d'intensité, s'élève à 45.

Le pic secondaire P'2 se distingue plus aisément que la bosse P'1, de l'essai précédent, ce qui témoigne d'un meilleur contraste de l'image réalisée en utilisant la source de lumière blanche.

L'histogramme correspondant au troisième essai (source de lumière dont la plage spectrale s'étend entre 538 nm et 552 nm), portant la référence 3 sur la figure 6, comporte deux pics P3 et P'3 bien délimités, correspondant respectivement aux agglutinats et aux zones claires. Le pic P3 est maximal au niveau d'intensité de 71, et s'étend dans la plage d'intensité [50-110]. Le pic P'3 est maximal au niveau d'intensité 248, et s'étend sur la plage d'intensité remarquablement étroite [246 -250].

Sur la base de cet histogramme, les zones sombres et les zones claires de l'image 4C peuvent être délimitées en considérant respectivement les pixels dont l'intensité est inférieure à 110 (seuil bas) et les pixels sont l'intensité est supérieure à 246 (seuil haut). L'écart entre le seuil bas et le seuil haut, exprimé en valeur d'intensité, s'élève à 113.

Les sommets des pics correspondant respectivement aux agglutinats et aux zones claires sont plus espacés lorsqu'on utilise la source de lumière du troisième essai, ce qui témoigne d'une image mieux contrastée, permettant une information quantitative plus précise quant à l'état d'agglutination des particules.

Le procédé précédemment décrit pourra être utilisé pour caractériser l'agglutination de particules, et en particulier des globules rouges, dans un liquide corporel ou dans un liquide obtenu à partir d'un liquide corporel. Le liquide corporel peut être du sang, mais également d'autres liquides susceptibles de comporter des particules pouvant s'agglutiner en particulier des globules rouges : liquide céphalorachidien, urine...

Les particules peuvent être des globules rouges, ou autres cellules dont la transmission optique est comparable. Il peut également s'agir de gouttelettes non miscibles avec le milieu 14, par exemple des gouttelettes lipidiques.

Le procédé peut être mis en oeuvre dans le domaine du diagnostic médical, pour le dosage d'un analyte dans un liquide corporel, ou pour les applications de détermination du groupe sanguin.

## Revendications

1. Procédé de détermination d'un niveau d'agglutination dans un échantillon (10), l'échantillon (10) comportant des particules (1, 2, 3, 4, 5, 6, 7, 8, 9), lesdites particules étant aptes à s'agglutiner de manière à former un ou une pluralité d'agglutinats (1-2-3-4, 5-6-7-8-9) dans ledit échantillon, le procédé comportant les étapes suivantes :
- illumination dudit échantillon à l'aide d'une source de lumière (11), la source de lumière produisant une onde lumineuse incidente (12), se propageant vers l'échantillon (10) selon un axe de propagation (Z) ;
- acquisition, à l'aide d'un photodétecteur matriciel (16) d'une image (I) de l'échantillon, l'échantillon étant disposé entre ladite source de lumière et ledit photodétecteur matriciel ;
- détermination d'un niveau d'agglutination de particules dans l'échantillon, à l'aide de ladite image ;
**caractérisé en ce que** :
- ladite onde lumineuse incidente (12) présente une bande spectrale centrée sur une longueur d'onde (λₑₘ) inférieure à 600 nm.

2. Procédé selon la revendication 1, dans lequel les particules sont des globules rouges.

3. Procédé selon l'une quelconque des revendications précédentes, dans lequel l'échantillon (10) comporte un liquide corporel (14).

4. Procédé selon la revendication 3, dans lequel le liquide corporel (14) est du sang.

5. Procédé selon l'une quelconque des revendications précédentes, dans ladite bande spectrale est centrée sur une longueur d'onde (λₑₘ) comprise entre 400 nm et 600 nm.

6. Procédé selon l'une quelconque des revendications précédentes, dans lequel la largeur de ladite bande spectrale est inférieure à 200 nm ou est inférieure à 100 nm.

7. Procédé selon l'une quelconque des revendications précédentes, dans lequel l'échantillon présente une épaisseur (ε), selon ledit axe de propagation (Z), inférieure à 1cm.

8. Procédé selon l'une quelconque des revendications précédentes, dans lequel la détermination du niveau d'agglutination comporte les étapes suivantes :
i) sélection, dans ladite image (I), de zones sombres, dont l'intensité est inférieure à un premier seuil et/ou de zones claires, dont l'intensité est supérieure à un deuxième seuil ;
ii) détermination d'un indicateur statistique représentatif desdites zones sélectionnées ;
iii) détermination d'un niveau d'agglutination en fonction de la valeur dudit indicateur.

9. Procédé selon l'une quelconque des revendications précédentes, comportant, préalablement à l'acquisition de ladite image, une étape d'ajout d'un réactif (17) dans l'échantillon, apte à engendrer l'agglutination des particules.

10. Procédé selon l'une quelconque des revendications précédentes, dans lequel il n'y a pas de d'optique de grossissement entre l'échantillon (10) et le photodétecteur matriciel (16).

11. Dispositif de détermination d'un niveau d'agglutination dans un échantillon (10), l'échantillon comportant des particules (1,2,3,4,5,6,7,8,9), lesdites particules étant aptes à s'agglutiner de manière à former un ou une pluralité d'agglutinats (1-2-3-4, 5-6-7-8-9) dans ledit échantillon (10), le dispositif comprenant :
- une source de lumière (11) agencée pour produire une onde lumineuse incidente (12), selon un axe de propagation (Z), en direction dudit échantillon (10),
- un support, pour maintenir l'échantillon (10) entre ladite source de lumière (11) et un photodétecteur matriciel (16),
- le photodétecteur matriciel (16), étant agencé pour acquérir une image (I) de l'onde lumineuse (22) transmise par l'échantillon, lorsque ce dernier est exposé à ladite onde lumineuse incidente (12),
- un processeur (20), apte à déterminer un niveau d'agglutination des particules dans l'échantillon en fonction de ladite image (I),
le dispositif étant **caractérisé en ce que** :
- la source de lumière (11) est configurée de telle sorte que ladite onde lumineuse (12) incidente présente une bande spectrale centrée sur une longueur d'onde (λₑₘ) inférieure à 600 nm.

12. Dispositif selon la revendication 11, dans lequel ladite bande spectrale est centrée sur une longueur d'onde (λem) comprise entre 440 nm et 600 nm.

13. Dispositif selon l'une quelconque des revendications 11 ou 12, dans lequel la largeur de ladite bande spectrale est inférieure à 200 nm ou est inférieure à 100 nm.

14. Dispositif selon l'une quelconque des revendications 11 à 13, dans lequel il n'y a pas d'optique de grossissement entre le support de l'échantillon et le photodétecteur matriciel (16).
